# EUROPÄISCHE PATENTSCHRIFT

(11) **EP 1 006 192 B1**
(45) Veröffentlichungstag und Bekanntmachung des Hinweises auf die Patenterteilung: **09.09.2009**
(21) Anmeldenummer: 99122650.7
(22) Anmeldetag: 13.11.1999
(51) Int. Cl.: C12N 15/60, C12N 15/54, C12N 15/77, C12P 13/02, C12N 9/88

(54) **Verfahren zur fermentativen Herstellung von D-Pantothensäure durch Verstärkung des panD-Gens in Mikroorganismen**
Method for the fermentative production of D- pantothenic acid by amplification of the panD gene of microorganisms
Procédé pour la préparation d'acide pantothénique par amplification du gène panD de microorganismes

(30) Priorität: 01.12.1998 DE 19855313
(43) Veröffentlichungstag der Anmeldung: 07.06.2000
(73) Patentinhaber: Evonik Degussa GmbH, 45128 Essen (DE)
(72) Erfinder: Dusch, Nicole, Dr., 33619 Bielefeld (DE); Kalinowski, Jörn, Dr., 33615 Bielefeld (DE); Pühler, Alfred, Prof. Dr., 33739 Bielefeld (DE)

(56) Entgegenhaltungen:
- EP-A- 0 590 857
- EP-A- 1 001 027
- EP-A- 1 006 193
- EP-A- 1 083 225
- WO-A-01/00843
- US-A- 5 518 906
- DUSCH N ET AL: "EXPRESSION OF THE CORYNEBACTERIUM GLUTAMICUM PAND GENE ENCODING L-ASPARTATE-ALPHA-DECARBOXYLASE LEAD: TO PANTOTHENATE OVERPRODUCTION IN ESCHERICHIA COLI" APPLIED AND ENVIRONMENTAL MICROBIOLOGY, WASHINGTON,DC, US, Bd. 65, Nr. 4, April 1999 (1999-04), Seiten 1530-1539, XP000930097 ISSN: 0099-2240
- SAHM HERMANN ET AL: "D-pantothenate synthesis in Corynebacterium glutamicum and use of panBC and genes encoding L-valine synthesis for D-pantothenate overproduction" APPLIED AND ENVIRONMENTAL MICROBIOLOGY, WASHINGTON,DC, US, Bd. 65, Nr. 5, Mai 1999 (1999-05), Seiten 1973-1979, XP002138605 ISSN: 0099-2240

### SEQUENZPROTOKOLL

(1) ALLGEMEINE ANGABEN:
   (i) ANMELDER:
      (A) NAME: Degussa Aktiengesellschaft
      (B) STRASSE: Weissfrauenstr. 9
      (C) ORT: Frankfurt am Main
      (D) BUNDESLAND: Hessen
      (E) LAND: Deutschland
      (F) POSTLEITZAHL: D-60311
   (ii) BEZEICHNUNG DER ERFINDUNG: Verfahren zur fermentativen Herstellung von D-Pantothensaeure durch Verstaerkung des panD-Gens in Mikroorganismen
   (iii) ANZAHL DER SEQUENZEN: 5
   (iv) COMPUTER-LESBARE FASSUNG:
      (A) DATENTRAEGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LAENGE: 540 Basenpaare
      (B) ART: Nucleotid
      (C) STRANGFORM: Doppelstrang
      (D) TOPOLOGIE: linear
   (ii) ART DES MOLEKUELS: Genom-DNA
   (iii) HYPOTHETISCH: NEIN
   (iv) ANTISENSE: NEIN
   (vi) URSPRUENGLICHE HERKUNFT:
      (A) ORGANISMUS: Corynebacterium glutamicum
      (B) STAMM: ATCC13032
   (ix) MERKMAL:
      (A) NAME/SCHLUESSEL: CDS
      (B) LAGE:77..484
      (D) SONSTIGE ANGABEN:/codon_start= 77 /EC_number= 4.1.1.11 /product= "L-Aspartat-1-decarboxylase" /gene= "panD"
   (xi) SEQUENZBESCHREIBUNG: SEQ ID NO: 1:
   AATATTCCTT TCCTTGTCAT CTCACGCTAT GATTTCTAAA ACTTGCAGGA CAACCCCCAT       60
   AAGGACACCA CAGGAC ATG CTG CGC ACC ATC CTC GGA AGT AAG ATT CAC          109
                     Met Leu Arg Thr Ile Leu Gly Ser Lys Ile His
                      1               5                  10
   CGA GCC ACT GTC ACT CAA GCT GAT CTA GAT TAT GTT GGC TCT GTA ACC        157
   Arg Ala Thr Val Thr Gln Ala Asp Leu Asp Tyr Val Gly Ser Val Thr
               15                  20                  25
   ATC GAC GCC GAC CTG GTT CAC GCC GCC GGA TTG ATC GAA GGC GAA AAA        205
   Ile Asp Ala Asp Leu Val His Ala Ala Gly Leu Ile Glu Gly Glu Lys
           30                  35                  40
   GTT GCC ATC GTA GAC ATC ACC AAC GGC GCT CGT CTG GAA ACT TAT GTC        253
   Val Ala Ile Val Asp Ile Thr Asn Gly Ala Arg Leu Glu Thr Tyr Val
       45                  50                  55
   ATT GTG GGC GAC GCC GGA ACG GGC AAT ATT TGC ATC AAT GGT GCC GCT        301
   Ile Val Gly Asp Ala Gly Thr Gly Asn Ile Cys Ile Asn Gly Ala Ala
   60                  65                  70                  75
   GCA CAC CTT ATT AAT CCT GGC GAT CTT GTG ATC ATC ATG AGC TAC CTT        349
   Ala His Leu Ile Asn Pro Gly Asp Leu Val Ile Ile Met Ser Tyr Leu
                   80                  85                  90
   CAG GCA ACT GAT GCG GAA GCC AAG GCG TAT GAG CCA AAG ATT GTG CAC        397
   Gln Ala Thr Asp Ala Glu Ala Lys Ala Tyr Glu Pro Lys Ile Val His
               95                 100                 105
   GTG GAC GCC GAC AAC CGC ATC GTT GCG CTC GGC AAC GAT CTT GCG GAA        445
   Val Asp Ala Asp Asn Arg Ile Val Ala Leu Gly Asn Asp Leu Ala Glu
           110                 115                 120
   GCA CTA CCT GGA TCC GGG CTT TTG ACG TCG AGA AGC ATT TAGCGTTTTA         494
   Ala Leu Pro Gly Ser Gly Leu Leu Thr Ser Arg Ser Ile
       125                 130                 135
   GCTCGCCAAT ATTGCTGCCG GCCTCGTTGA AAATGGTCAT GGTGGC                     540
(2) ANGABEN ZU SEQ ID NO: 2:
   (i) SEQUENZKENNZEICHEN:
      (A) LAENGE: 136 Aminosaeuren
      (B) ART: Aminosaeure
      (D) TOPOLOGIE: linear
   (ii) ART DES MOLEKUELS: Protein
   (xi) SEQUENZBESCHREIBUNG: SEQ ID NO: 2:
   Met Leu Arg Thr Ile Leu Gly Ser Lys Ile His Arg Ala Thr Val Thr
    1               5                  10                  15
   Gln Ala Asp Leu Asp Tyr Val Gly Ser Val Thr Ile Asp Ala Asp Leu
               20                  25                  30
   Val His Ala Ala Gly Leu Ile Glu Gly Glu Lys Val Ala Ile Val Asp
           35                  40                  45
   Ile Thr Asn Gly Ala Arg Leu Glu Thr Tyr Val Ile Val Gly Asp Ala
       50                  55                  60
   Gly Thr Gly Asn Ile Cys Ile Asn Gly Ala Ala Ala His Leu Ile Asn
   65                  70                  75                  80
   Pro Gly Asp Leu Val Ile Ile Met Ser Tyr Leu Gln Ala Thr Asp Ala
                   85                  90                  95
   Glu Ala Lys Ala Tyr Glu Pro Lys Ile Val His Val Asp Ala Asp Asn
               100                 105                 110
   Arg Ile Val Ala Leu Gly Asn Asp Leu Ala Glu Ala Leu Pro Gly Ser
           115                 120                 125
   Gly Leu Leu Thr Ser Arg Ser Ile
       130                 135
(2) ANGABEN ZU SEQ ID NO: 3:
   (i) SEQUENZKENNZEICHEN:
      (A) LAENGE: 2164 Basenpaare
      (B) ART: Nucleotid
      (C) STRANGFORM: Doppelstrang
      (D) TOPOLOGIE: linear
   (ii) ART DES MOLEKUELS: Genom-DNA
   (iii) HYPOTHETISCH: NEIN
   (iv) ANTISENSE: NEIN
   (vi) URSPRUENGLICHE HERKUNFT:
      (A) ORGANISMUS: Corynebacterium glutamicum
      (B) STAMM: ATCCl3032
   (ix) MERKMAL:
      (A) NAME/SCHLUESSEL: CDS
      (B) LAGE:351..1163
      (D) SONSTIGE ANGABEN:/codon_start= 351 /EC_number= 4.1.2.12 /product= "Ketopantoathydroxymethyltransferase" /gene= "panB"
   (ix) MERKMAL:
      (A) NAME/SCHLUESSEL: CDS
      (B) LAGE:1166..2002
      (D) SONSTIGE ANGABEN:/codon_start= 1166 /EC_number= 6.3.2.1 /product= "Pantothenatsynthetase" /gene= "panC"
   (xi) SEQUENZBESCHREIBUNG: SEQ ID NO: 3:
   GCTTCGGGGT ACCAATTCCT TTAAGAACCA TCAGATCAAT CTGTTGTACA TTCTCGGCCA      60
   GATTCAGCTT TTCGGTAAGG ACGAAACACT TTCACTTGAA TCGGCAGCAA AGTTTCTTAA     120
   AGTTTCTAAG GCAACTGCAA CGAGGTATTT TAGAACTCTC CGAGAAATGG AATTAGTTCA     180
   CGAGGTCAGC AAACGCCCTT TGCGGTTTGC GCTCACGGAT AAAGGTCGTG AGATAGTAGG     240
   TCTTGAGGTA AAAATTTGAC TCCATAACGA GAACTTAATC GAGCAACACC CCTGAACAGT     300
   GAATCAAATC GGAATTTATT TATTCTGAGC TGGTCATCAC ATCTATACTC ATG CCC        356
                                                          Met Pro
   ATG TCA GGC ATT GAT GCA AAG AAA ATC CGC ACC CGT CAT TTC CGC GAA      404
   Met Ser Gly Ile Asp Ala Lys Lys Ile Arg Thr Arg His Phe Arg Glu
       140                 145                 150
   GCT AAA GTA AAC GGC CAG AAA GTT TCG GTT CTC ACC AGC TAT GAT GCG      452
   Ala Lys Val Asn Gly Gln Lys Val Ser Val Leu Thr Ser Tyr Asp Ala
   155                 160                 165                 170
   CTT TCG GCG CGC ATT TTT GAT GAG GCT GGC GTC GAT ATG CTC CTT GTT      500
   Leu Ser Ala Arg Ile Phe Asp Glu Ala Gly Val Asp Met Leu Leu Val
                   175                 180                 185
   GGT GAT TCC GCT GCC AAC GTT GTG CTG GGT CGC GAT ACC ACC TTG TCG      548
   Gly Asp Ser Ala Ala Asn Val Val Leu Gly Arg Asp Thr Thr Leu Ser
               190                 195                 200
   ATC ACC TTG GAT GAG ATG ATT GTG CTG GCC AAG GCG GTG ACG ATC GCT      596
   Ile Thr Leu Asp Glu Met Ile Val Leu Ala Lys Ala Val Thr Ile Ala
           205                 210                 215
   ACG AAG CGT GCG CTT GTG GTG GTT GAT CTG CCG TTT GGT ACC TAT GAG      644
   Thr Lys Arg Ala Leu Val Val Val Asp Leu Pro Phe Gly Thr Tyr Glu
       220                 225                 230
   GTG AGC CCA AAT CAG GCG GTG GAG TCC GCG ATC CGG GTC ATG CGT GAA      692
   Val Ser Pro Asn Gln Ala Val Glu Ser Ala Ile Arg Val Met Arg Glu
   235                 240                 245                 250
   ACG GGT GCG GCT GCG GTG AAG ATC GAG GGT GGC GTG GAG ATC GCG CAG      740
   Thr Gly Ala Ala Ala Val Lys Ile Glu Gly Gly Val Glu Ile Ala Gln
                   255                 260                 265
   ACG ATT CGA CGC ATT GTT GAT GCT GGA ATT CCG GTT GTC GGC CAC ATC      788
   Thr Ile Arg Arg Ile Val Asp Ala Gly Ile Pro Val Val Gly His Ile
               270                 275                 280
   GGG TAC ACC CCG CAG TCC GAG CAT TCC TTG GGC GGC CAC GTG GTT CAG      836
   Gly Tyr Thr Pro Gln Ser Glu His Ser Leu Gly Gly His Val Val Gln
           285                 290                 295
   GGT CGT GGC GCG AGT TCT GGA AAG CTC ATC GCC GAT GCC CGC GCG TTG      884
   Gly Arg Gly Ala Ser Ser Gly Lys Leu Ile Ala Asp Ala Arg Ala Leu
       300                 305                 310
   GAG CAG GCG GGT GCG TTT GCG GTT GTG TTG GAG ATG GTT CCA GCA GAG      932
   Glu Gln Ala Gly Ala Phe Ala Val Val Leu Glu Met Val Pro Ala Glu
   315                 320                 325                 330
   GCA GCG CGC GAG GTT ACC GAG GAT CTT TCC ATC ACC ACT ATC GGA ATC      980
   Ala Ala Arg Glu Val Thr Glu Asp Leu Ser Ile Thr Thr Ile Gly Ile
                   335                 340                 345
   GGT GCC GGC AAT GGC ACA GAT GGG CAG GTT TTG GTG TGG CAG GAT GCC     1028
   Gly Ala Gly Asn Gly Thr Asp Gly Gln Val Leu Val Trp Gln Asp Ala
               350                 355                 360
   TTC GGC CTC AAC CGC GGC AAG AAG CCA CGC TTC GTC CGC GAG TAC GCC     1076
   Phe Gly Leu Asn Arg Gly Lys Lys Pro Arg Phe Val Arg Glu Tyr Ala
           365                 370                 375
   ACC TTG GGC GAT TCC TTG CAC GAC GCC GCG CAG GCC TAC ATC GCC GAT     1124
   Thr Leu Gly Asp Ser Leu His Asp Ala Ala Gln Ala Tyr Ile Ala Asp
       380                 385                 390
   ATC CAC GCG GGT ACC TTC CCA GGC GAA GCG GAG TCC TTT TA ATG CAG      1171
   Ile His Ala Gly Thr Phe Pro Gly Glu Ala Glu Ser Phe    Met Gln
   395                 400                 405              1
   GTA GCA ACC ACA AAG CAG GCG CTT ATC GAC GCC CTC CTC CAC CAC AAA     1219
   Val Ala Thr Thr Lys Gln Ala Leu Ile Asp Ala Leu Leu His His Lys
             5                  10                  15
   TCC GTC GGG CTC GTC CCC ACC ATG GGT GCG CTA CAC AGC GGA CAC GCC     1267
   Ser Val Gly Leu Val Pro Thr Met Gly Ala Leu His Ser Gly His Ala
        20                  25                  30
   TCG TTG GTT AAA GCA GCA CGC GCT GAA AAC GAC ACT GTT GTA GCC AGT     1315
   Ser Leu Val Lys Ala Ala Arg Ala Glu Asn Asp Thr Val Val Ala Ser
    35                  40                  45                  50
   ATT TTT GTC AAT CCC CTG CAG TTT GAA GCA CTC GGT GAT TGC GAT GAT     1363
   Ile Phe Val Asn Pro Leu Gln Phe Glu Ala Leu Gly Asp Cys Asp Asp
                    55                  60                  65
   TAC CGC AAC TAT CCC CGC CAA CTC GAC GCC GAT TTA GCA CTG CTT GAA     1411
   Tyr Arg Asn Tyr Pro Arg Gln Leu Asp Ala Asp Leu Ala Leu Leu Glu
                70                  75                  80
   GAG GCA GGT GTG GAT ATT GTG TTC GCA CCC GAT GTG GAG GAA ATG TAC     1459
   Glu Ala Gly Val Asp Ile Val Phe Ala Pro Asp Val Glu Glu Met Tyr
            85                  90                  95
   CCC GGT GGC TTG CCA CTA GTG TGG GCG CGC ACC GGT TCC ATC GGA ACA     1507
   Pro Gly Gly Leu Pro Leu Val Trp Ala Arg Thr Gly Ser Ile Gly Thr
       100                 105                 110
   AAA TTG GAG GGT GCC AGC AGG CCT GGC CAT TTC GAT GGT GTG GCT ACC     1555
   Lys Leu Glu Gly Ala Ser Arg Pro Gly His Phe Asp Gly Val Ala Thr
   115                 120                 125                 130
   GTG GTG GCG AAG CTG TTC AAT TTG GTG CGC CCT GAT CGT GCA TAT TTT     1603
   Val Val Ala Lys Leu Phe Asn Leu Val Arg Pro Asp Arg Ala Tyr Phe
                   135                 140                 145
   GGA CAA AAA GAT GCT CAG CAG GTT GCG GTG ATT CGG CGA TTG GTT GCC     1651
   Gly Gln Lys Asp Ala Gln Gln Val Ala Val Ile Arg Arg Leu Val Ala
               150                 155                 160
   GAT CTA GAC ATT CCC GTG GAG ATT CGT CCC GTT CCG ATT ATT CGT GGC     1699
   Asp Leu Asp Ile Pro Val Glu Ile Arg Pro Val Pro Ile Ile Arg Gly
           165                 170                 175
   GCC GAT GGC TTA GCC GAA TCC AGC CGC AAT CAA CGT CTT TCT GCG GAT     1747
   Ala Asp Gly Leu Ala Glu Ser Ser Arg Asn Gln Arg Leu Ser Ala Asp
       180                 185                 190
   CAG CGA GCG CAA GCT CTG GTG CTG CCG CAG GTG TTG AGT GGG TTG CAG     1795
   Gln Arg Ala Gln Ala Leu Val Leu Pro Gln Val Leu Ser Gly Leu Gln
   195                 200                 205                 210
   CGT CGA AAA GCA GCT GGT GAA GCG CTA GAT ATC CAA GGT GCG CGC GAC     1843
   Arg Arg Lys Ala Ala Gly Glu Ala Leu Asp Ile Gln Gly Ala Arg Asp
                   215                 220                 225
   ACC TTG GCC AGC GCC GAC GGC GTG CGC TTG GAT CAC CTG GAA ATT GTC     1891
   Thr Leu Ala Ser Ala Asp Gly Val Arg Leu Asp His Leu Glu Ile Val
               230                 235                 240
   GAT CCA GCC ACC CTC GAA CCA TTA GAA ATC GAC GGC CTG CTC ACC CAA     1939
   Asp Pro Ala Thr Leu Glu Pro Leu Glu Ile Asp Gly Leu Leu Thr Gln
           245                 250                 255
   CCA GCG TTG GTG GTC GGC GCG ATT TTC GTG GGG CCG GTG CGG TTG ATC       1987
   Pro Ala Leu Val Val Gly Ala Ile Phe Val Gly Pro Val Arg Leu Ile
       260                 265                 270
   GAC AAT ATC GAG CTC TAGTACCAAC CCTGCGTTGC AGCACGCAGC TTCGCATAAC       2042
   Asp Asn Ile Glu Leu
   275
   GCGTGCTCAG CTCAGTGTTT TTAGGTGCGC GGTGCGGATC GGAACCGGGA GTTGGCCACT     2102
   GCGGTGGCGT GGCCTCACCC GACAGCGCCC ATGCCGCCTG ACGAGCTGCA CCCAACGCCA     2162
   CA                                                                    2164
(2) ANGABEN ZU SEQ ID NO: 4:
   (i) SEQUENZKENNZEICHEN:
      (A) LAENGE: 271 Aminosaeuren
      (B) ART: Aminosaeure
      (D) TOPOLOGIE: linear
   (ii) ART DES MOLEKUELS: Protein
   (xi) SEQUENZBESCHREIBUNG: SEQ ID NO: 4:
   Met Pro Met Ser Gly Ile Asp Ala Lys Lys Ile Arg Thr Arg His Phe
    1               5                  10                  15
   Arg Glu Ala Lys Val Asn Gly Gln Lys Val Ser Val Leu Thr Ser Tyr
               20                  25                  30
   Asp Ala Leu Ser Ala Arg Ile Phe Asp Glu Ala Gly Val Asp Met Leu
           35                  40                  45
   Leu Val Gly Asp Ser Ala Ala Asn Val Val Leu Gly Arg Asp Thr Thr
       50                  55                  60
   Leu Ser Ile Thr Leu Asp Glu Met Ile Val Leu Ala Lys Ala Val Thr
   65                  70                  75                  80
   Ile Ala Thr Lys Arg Ala Leu Val Val Val Asp Leu Pro Phe Gly Thr
                   85                  90                  95
   Tyr Glu Val Ser Pro Asn Gln Ala Val Glu Ser Ala Ile Arg Val Met
               100                 105                 110
   Arg Glu Thr Gly Ala Ala Ala Val Lys Ile Glu Gly Gly Val Glu Ile
           115                 120                 125
   Ala Gln Thr Ile Arg Arg Ile Val Asp Ala Gly Ile Pro Val Val Gly
       130                 135                 140
   His Ile Gly Tyr Thr Pro Gln Ser Glu His Ser Leu Gly Gly His Val
   145                 150                 155                 160
   Val Gln Gly Arg Gly Ala Ser Ser Gly Lys Leu Ile Ala Asp Ala Arg
                   165                 170                 175
   Ala Leu Glu Gln Ala Gly Ala Phe Ala Val Val Leu Glu Met Val Pro
               180                 185                 190
   Ala Glu Ala Ala Arg Glu Val Thr Glu Asp Leu Ser Ile Thr Thr Ile
           195                 200                 205
   Gly Ile Gly Ala Gly Asn Gly Thr Asp Gly Gln Val Leu Val Trp Gln
       210                 215                 220
   Asp Ala Phe Gly Leu Asn Arg Gly Lys Lys Pro Arg Phe Val Arg Glu
   225                 230                 235                 240
   Tyr Ala Thr Leu Gly Asp Ser Leu His Asp Ala Ala Gln Ala Tyr Ile
                   245                 250                 255
   Ala Asp Ile His Ala Gly Thr Phe Pro Gly Glu Ala Glu Ser Phe
               260                 265                 270
(2) ANGABEN ZU SEQ ID NO: 5:
   (i) SEQUENZKENNZEICHEN:
      (A) LAENGE: 279 Aminosaeuren
      (B) ART: Aminosaeure
      (D) TOPOLOGIE: linear
   (ii) ART DES MOLEKUELS: Protein
   (xi) SEQUENZBESCHREIBUNG: SEQ ID NO: 5:
   Met Gln Val Ala Thr Thr Lys Gln Ala Leu Ile Asp Ala Leu Leu His
    1               5                  10                  15
   His Lys Ser Val Gly Leu Val Pro Thr Met Gly Ala Leu His Ser Gly
               20                  25                  30
   His Ala Ser Leu Val Lys Ala Ala Arg Ala Glu Asn Asp Thr Val Val
               35                  40                  45
   Ala Ser Ile Phe Val Asn Pro Leu Gln Phe Glu Ala Leu Gly Asp Cys
       50                  55                  60
   Asp Asp Tyr Arg Asn Tyr Pro Arg Gln Leu Asp Ala Asp Leu Ala Leu
   65                  70                  75                  80
   Leu Glu Glu Ala Gly Val Asp Ile Val Phe Ala Pro Asp Val Glu Glu
                   85                  90                  95
   Met Tyr Pro Gly Gly Leu Pro Leu Val Trp Ala Arg Thr Gly Ser Ile
               100                 105                 110
   Gly Thr Lys Leu Glu Gly Ala Ser Arg Pro Gly His Phe Asp Gly Val
           115                 120                 125
   Ala Thr Val Val Ala Lys Leu Phe Asn Leu Val Arg Pro Asp Arg Ala
       130                 135                 140
   Tyr Phe Gly Gln Lys Asp Ala Gln Gln Val Ala Val Ile Arg Arg Leu
   145                 150                 155                 160
   Val Ala Asp Leu Asp Ile Pro Val Glu Ile Arg Pro Val Pro Ile Ile
                   165                 170                 175
   Arg Gly Ala Asp Gly Leu Ala Glu Ser Ser Arg Asn Gln Arg Leu Ser
               180                 185                 190
   Ala Asp Gln Arg Ala Gln Ala Leu Val Leu Pro Gln Val Leu Ser Gly
           195                 200                 205
   Leu Gln Arg Arg Lys Ala Ala Gly Glu Ala Leu Asp Ile Gln Gly Ala
       210                 215                 220
   Arg Asp Thr Leu Ala Ser Ala Asp Gly Val Arg Leu Asp His Leu Glu
   225                 230                 235                 240
   Ile Val Asp Pro Ala Thr Leu Glu Pro Leu Glu Ile Asp Gly Leu Leu
                   245                 250                 255
   Thr Gln Pro Ala Leu Val Val Gly Ala Ile Phe Val Gly Pro Val Arg
               260                 265                 270
   Leu Ile Asp Asn Ile Glu Leu
           275

## Beschreibung

### Stand der Technik

Die Pantothensäure stellt ein kommerziell bedeutendes Vitamin dar, das in der Kosmetik, der Medizin, der Humanernährung und in der Tierernährung Anwendung findet.

Pantothensäure kann durch chemische Synthese oder biotechnisch durch Fermentation geeigneter Mikroorganismen in geeigneten Nährlösungen hergestellt werden. Bei der chemischen Synthese ist das DL-Pantolacton eine wichtige Zwischenstufe. Es wird in einem mehrstufigen Verfahren aus Formaldehyd, Isobutylaldehyd und Cyanid hergestellt. In weiteren Verfahrensschritten wird das racemische Gemisch aufgetrennt und D-Pantolacton mit β-Alanin kondensiert und man erhält D-Pantothensäure.

Der Vorteil der fermentativen Herstellung durch Mikroorganismen liegt in der direkten Bildung der gewünschten stereoisomeren D-Form die frei von L-Pantothensäure ist.

Verschiedene Arten von Bakterien, wie z. B. Escherichia coli, Arthrobacter ureafaciens, Corynebacterium erythrogenes, Brevibacterium ammoniagenes und auch Hefen, wie z. B. Debaromyces castellii können wie in EP-A 0 493 060 gezeigt, in einer Nährlösung, die Glucose, DL-Pantoinsäure und β-Alanin enthält, D-Pantothensäure produzieren. EP-A 0 493 060 zeigt weiterhin, daß bei Escherichia coli durch Amplifikation von Pantothensäure-Biosynthesegenen aus E.coli, die auf den Plasmiden pFV3 und pFV5 enthalten sind, in einer Nährlösung, die Glucose, DL-Pantoinsäure und β-Alanin enthält, die Bildung von D-Pantothensäure verbessert wird.

EP-A 0 590 857 und US-Patent 5,518,906 beschreiben von Escherichia coli Stamm IFO3547 abgeleitete Mutanten wie FV5714, FV525, FV814, FV521, FV221, FV6051 und FV5069 die Resistenzen gegen verschiedene Antimetabolite wie Salizylsäure, α-Ketobuttersäure, β-Hydroxyasparaginsäure, O-Methylthreonin und α-Ketoisovaleriansäure tragen und in einer Nährlösung, die Glucose enthält Pantoinsäure und in einer Nährlösung, die Glucose und β-Alanin enthält, D-Pantothensäure produzieren. In EPA 0 590 857 und US-Patent 5,518,906 wird weiterhin gezeigt, daß nach Amplifikation der Pantothensäure-Biosynthesegene, die auf dem Plasmid pFV31 enthalten sind, in den oben genannten Stämmen in einer Nährlösung die Glucose enthält die Produktion von D-Pantoinsäure und in einer Nährlösung die Glucose und β-Alanin enthält die Produktion von D-Pantothensäure verbessert wird.

In WO 97/10340 wird darüber hinaus gezeigt, daß in Pantothensäure bildenden Stämmen von Escherichia coli durch Erhöhung der Aktivität des Enzyms Acetohydroxysäure-Synthase II, einem Enzym der Valin Biosynthese, die Pantothensäure-Produktion weiter gesteigert werden kann.

### Aufgabe der Erfindung

Die Erfinder haben sich zur Aufgabe gestellt neue Grundlagen für verbesserte Verfahren zur fermentativen Herstellung von Pantothensäure bereitzustellen.

### Beschreibung der Erfindung

Das Vitamin Pantothensäure stellt ein kommerziell bedeutendes Produkt dar, das in der Kosmetik, der Medizin, der Humanernährung und in der Tierernährung Anwendung findet. Es besteht daher ein allgemeines Interesse daran neue Verfahren zur Herstellung von Pantothensäure bereitzustellen.

Wenn im Folgenden D-Pantothensäure oder Pantothensäure oder Pantothenat erwähnt werden, sind damit nicht nur die freien Säuren, sondern auch die Salze der D-Pantothensäure wie z.B. das Calcium-, Natrium-, Ammonium- oder Kaliumsalz gemeint.

Gegenstand der Erfindung ist unter anderem ein Verfahren zur fermentativen Herstellung von D-Pantothensäure unter Verwendung von Mikroorganismen, die insbesondere bereits D-Pantothensäure produzieren, und in denen das für die L-Aspartat-1-Decarboxylase (E.C. 4.1.1.11) kodierende panD-Gen einzeln oder in Kombination mit den Genen panB und/oder panC überexprimiert wird. Die Erfindung betrifft weiter Nukleotidsequenzen des pan D-gens, wie sie in den Ansprüchen niedergelegt sind. Gegenstand der Erfindung sind ebenso Verfahren zur fermentativen Herstellung von D-Pantothensäure, die unter Verwendung der verbesserten, D-Pantothensäure erzeugenden Mikroorgainsmen gemäß den Ansprüchen 3, 4 und 8 bis 14 und 18, 19 durchgeführt werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können Pantothensäure aus Glucose, Saccharose, Lactose, Fructose, Maltose, Melasse, Stärke, Cellulose oder aus Glycerin und Ethanol herstellen. Es kann sich um Pilze oder Hefen oder Gram-positive Bakterien z. B. der Gattung Corynebacterium oder um Gram-negative Bakterien wie z. B. die der Enterobacteriaceae handeln. Bei der Familie der Enterobacteriaceae ist besonders die Gattung Escherichia mit der Art Escherichia coli zu nennen. Innerhalb der Art Escherichia coli sind die sogenannten K-12 Stämme wie z. B. die Stämme MG1655 oder W3110 (Neidhard et al.: Escherichia coli and Salmonella. Cellular and Molecular Biology (ASM Press, Washington D.C.)) oder der Escherichia coli Wildtypstamm IFO3547 (Institut für Fermentation, Osaka, Japan) und davon abgeleitete Mutanten zu nennen. Bei der Gattung Corynebacterium ist insbesondere die Art Corynebacterium glutamicum zu nennen, die in der Fachwelt für ihre Fähigkeit bekannt ist Aminosäuren zu produzieren. Zu dieser Art gehören Wildtypstämme wie z. B. Corynebacterium glutamicum ATCC13032, Brevibacterium flavum ATCC14067, Corynebacterium melassecola ATCC17965 und davon abgeleitete Mutanten.

Die Erfinder fanden heraus, daß Mikroorganismen nach Überexpression des neuen für die L-Aspartat-1-Decarboxylase (E.C. 4.1.1.11) kodierenden panD-Gens, insbesondere aus Corynebacterium glutamicum in verbesserter Weise Pantothensäure produzieren.

Die Erfinder haben darüberhinaus herausgefunden, daß die Überexpression des panD-Gens sich in Stämmen vorteilhaft auswirkt, in denen zusätzlich die für Ketopantoathydroxymethyltransferase und Pantothenatsynthetase kodierenden Gene panB und panC einzeln oder gemeinsam überexprimiert vorliegen.

Zur Erzielung einer Überexpression kann die Kopienzahl der entsprechenden Gene erhöht werden, oder es kann die Promotor- und Regulationsregion, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen D-Pantothenatbildung zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Jensen und Hammer (Biotechnology and Bioengineering 58, 191-195 (1998)) oder im Handbuch "Manual of Methods for General Bacteriology der American Society for Bacteriology (Washington D.C., USA, 1981). Darüberhinaus findet der Fachmann unter anderem Anleitungen bei Chang und Cohen (Journal of Bacteriology 134:1141-1156 (1978)), bei Hartley und Gregori (Gene 13:347-353 (1981)), bei Amann und Brosius (Gene 40:183-190 (1985)), bei de Broer et al. (Proceedings of the National of Sciences of the United States of America 80:21-25 (1983)), bei LaVallie et al. (BIO/TECHNOLOGY 11, 187-193 (1993)), in PCT/US97/13359, bei Llosa et al. (Plasmid 26:222-224 (1991)), bei Quandt und Klipp (Gene 80:161-169 (1989)), bei Hamilton (Journal of Bacteriology 171:4617-4622 (1989)), bei Makrides (Microbiological Reviews 60:512-538 (1996)) und in bekannten Lehrbüchern der Genetik und Molekularbiologie.

Zur Isolierung des panD-Gens oder anderer Gene wie z.B. der Gene panB und panC von C. glutamicum wird zunächst eine Genbank dieses Mikrorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 (Raleigh et al., 1988, Nucleic Acids Research 16:1563-1575) angelegt wurde. Zur Herstellung einer Genbank von C. glutamicum in E. coli können auch Plasmide wie pBR322 (Bolivar, Life Sciences, 25, 807-818 (1979)) oder pUC9 (Viera et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde.
Die Genbank wird anschließend in einen Indikatorstamm durch Transformation (Hanahan, Journal of Molecular Biology 166, 557-580, 1983) oder Elektroporation (Tauch et.al., 1994, FEMS Microbiological Letters, 123:343-347) eingebaut. Der Indikatorstamm zeichnet sich dadurch aus, dass er eine Mutation in dem interessierenden Gen besitzt, die einen detektierbaren Phänotyp z.B. eine Auxotrophie hervorruft. Im Rahmen der vorliegenden Erfindung ist die E. coli Mutante DV9 (Vallari und Rock, Journal of Bacteriology 1985, 164:136-142), die eine Mutation im panD-Gen trägt von besonderem Interesse. Ein anderes Beispiel für eine Pantothensäure-bedürftige E. coli Mutante ist der Stamm SJ2, der eine Mutation im panB-Gen trägt und vom Genetic Stock Center der Yale University (New Haven, Connecticut, USA) bezogen werden kann. Nach Transformation des Indikatorstammes wie z.B. der panD-Mutante DV9 mit einem rekombinanten Plasmid, welches das interessierende Gen wie z.B. das panD-Gen trägt und Expression des betreffenden Gens, wird der Indikatorstamm bezüglich der entsprechenden Eigenschaft wie z.B. der Pantothensäure-Bedürftigkeit prototroph. Das dergestalt isolierte Gen bzw. DNA-Fragment kann durch Bestimmung der Sequenz, wie z.B. bei Sanger et al. (Proceedings of the National of Sciences of the United States of America USA, 74:5463-5467, 1977) beschrieben, charakterisiert werden. Anschließend kann der Grad an Identität zu bekannten Genen, die in Datenbanken wie z.B. der GenBank (Benson et el., 1998, Nuleic Acids Research, 26:1-7) enthalten sind, mit publizierten Methoden (Altschul et al., 1990, Journal of Molecular Biology 215:403-410) analysiert werden.

Auf diese Weise wurde die neue für das Gen panD kodierende DNA-Sequenz von C. glutamicum erhalten, die als SEQ ID NO 1 Bestandteil der vorliegenden Erfindung ist. Weiterhin wurden aus der vorliegenden DNA-Sequenz mit den oben beschriebenen Methoden die Aminosäuresequenzen der entsprechenden Enzyme abgeleitet. In SEQ ID NO 2 ist die sich ergebende Aminosäuresequenz des panD-Genproduktes nämlich der L-Aspartat-1-Decarboxylase dargestellt. Weiterhin wurden auf diese Weise die für die Gene panB und panC kodierenden DNA-Sequenzen von C. glutamicum erhalten, die als SEQ ID NO 3 Bestandteil der vorliegenden Beschreibung ist. In SEQ ID NO 4 ist die sich ergebende Aminosäuresequenz des panB-Genproduktes nämlich der Ketopantoathydroxymethyltransferase dargestellt und in SEQ ID NO 5 die sich ergebende Aminosäuresequenz des panC-Genproduktes nämlich der Pantothenatsynthetase dargestellt.

Kodierende DNA-Sequenzen, die sich aus SEQ ID NO 1 durch die Degeneriertheit des genetischen Codes ergeben, sind ebenfalls Bestandteil der Erfindung. In gleicher Weise sind DNA-Sequenzen, die mit SEQ ID NO 1 und/oder SEQ ID NO 3 hybridisieren Bestandteil der Beschreibung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktikonsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2, SEQ ID NO 4 und/oder SEQ ID NO 5 ergeben sind ebenfalls Bestandteil der Erfindung.

Das auf diese Weise charakterisierte Gen kann anschließend einzeln oder in Kombination mit anderen in einem geeigneten Mikroorganismus zur Expression gebracht werden. Eine bekannte Methode Gene zu exprimieren bzw. überzuexprimieren besteht darin diese mit Hilfe von Plasmidvektoren zu amplifizieren, die überdies mit Expressionssignalen ausgestattet sein können. Als Plasmidvektoren kommen solche in Frage, die in den entsprechenden Mikroorganismen replizieren können. Für Escherichia coli kommen z.B. die Vektoren pSC101 (Vocke and Bastia, Proceedings of the National Academy of Sciences USA 80 (21), 6557-6561 (1983)) oder pKK223-3 (Brosius and Holy, Proceedings of the National Academy of Sciences USA 81, 6929 (1984)), für Corynebacterium glutamicum z.B. der Vektor pEKEx1 (Eikmanns et al., Gene 102:93-98 (1991)) oder pZ8-1 (Europäische Patentschrift 0 375 889) für die vorliegende Erfindung in Frage. Beispiele für derartige Mikroorganismen sind die C. glutamicum-Stämme ATCC13032/pND-D2 und ATCC13032/pND-DBC2 und der E. coli-Stamm MG1655/pND-D2, die die Plasmide pND-D2 und pND-DBC2 enthalten. Plasmid pND-D2 ist ein auf dem Plasmid pZ8-1 basierender E. coli-C. glutamicum Pendelvektor der das panD-Gen von C. glutamicum trägt. Plasmid pND-DBC2 ist ein auf dem Plasmid pZ8-1 basierender E. coli-C. glutamicum Pendelvektor der die Gene panD, panB und panC von C. glutamicum trägt.

Es ist dem Fachmann klar, daß chromosomale Mutationen, die Resistenz gegen Metabolite und Antimetabolite bewirken oder die das Abfliessen von Vorstufen der Pantothensäure verhindern, in vorteilhafter Weise mit den Massnahmen die Gegenstand der Erfindung sind kombiniert werden können.

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Pantothensäure-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies zur zusätzlichen Steigerung der Pantothensäure-Produktion Vorstufen der Pantothensäure wie Aspartat, β-Alanin, Ketoisovalerat, Ketopantoinsäure oder Pantoinsäure und gegebenenfalls deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 50°C und vorzugsweise bei 25°C bis 45°C. Die Kultur wird solange fortgesetzt bis sich ein Maximum an Pantothensäure gebildet hat. Dieses Ziel wird normalerweise innerhalb von 10 Stunden bis 160 Stunden erreicht. Anschließend wird de Pantothensäure isoliert.

Stämme, die eine hohe Aktivität des Enzyms L-Aspartat 1-Decarboxylase besitzen, können auch für die Herstellung von β-Alanin aus L-Aspartat eingesetzt werden. Hierzu können fermentative Verfahren, enzymatische Umwandlungsreaktionen oder Kombinationen beider eingesetzt werden.

Die Konzentration an gebildeter Pantothensäure kann mit bekannten Verfahren (Velisek; Chromatographic Science 60, 515-560 (1992)) bestimmt werden.

Folgende Mikroorganismen wurden bei der Deutschen Sammlung für Mikroorganismen und Zellkulturen (DSMZ, Braunschweig, Deutschland) gemäß Budapester Vertrag hinterlegt:
Corynebacterium glutamicum ATCC13032/pND-D2 als DSM12438
Corynebacterium glutamicum ATCC13032/pND-DBC2 als DSM12437

### Beispiele

Die vorliegende Erfindung wird im folgenden anhand von Ausführungsbeispielen näher erläutert.

### Beispiel 1

### Klonierung und Sequenzierung des panD-Gens von C. glutamicum

### 1. Klonierung des panD-Gens

Chromosomale DNA aus C. glutamicum ATCC 13032 wurde wie bei Tauch et al. (1995, Plasmid, 33:168-179) beschrieben, isoliert und mit der Restriktionsenzym Sau3A (Pharmacia Biotech (Freiburg, Deutschland), Produktbeschreibung Sau3A, Code no. 27-0913-02) partiell gespalten. DNA-Fragmente in einem Größenbereich von 7-9 kb wurden mit Hilfe des "Nucleotrap Extraction Kit for Nucleic Acids" (Macherey und Nagel, Düren, Deutschland; Cat. No. 740584) isoliert und in die dephosphorylierte BamHI-Schnittstelle des Vektors pUC19 (Norrander et al., 1982, Gene, 26:101-106), der von der Firma MBI Fermentas (Vilnius,Litauen) bezogen wurde, ligiert. Die Ligation wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Acadamies of Sciences USA, 87:4645-4649) elektroporiert (Tauch, 1994, FEMS Microbiological Letters, 123:343-347) und auf LB-Agar (Lennox, 1955, Virology, 1:190) + 100 µg/ml Ampicillin ausplattiert. Nach Inkubation für 24 Stunden bei 37°C konnte die C. glutamicum Genbank durch Reisolierung der Plasmid-DNA nach der "Alkalischen-Lyse-Methode" von Birnboim und Doly (Nucleic Acids Research, 7: 1513-1523, 1997) aus den Transformanten gewonnen werden. Mit dieser Genbank wurden kompetente Zellen des E. coli Stamms DV9 (Vallari und Rock, 1985, Journal of Bacteriology, 164:136-142), welcher eine Mutation im panD-Gen trägt, elektroporiert. Der Elektroporationsansatz wurde im Anschluß an die Regenerationsphase (Tauch et.al., 1994, FEMS Microbiological Letters, 123:343-347) zweimal mit Medium E (Vogel and Bonner, 1956, Journal of Biolgical Chemistry, 218:97-106) gewaschen. Die Zusammensetzung des Mediums E ist in Tabelle 1 dargestellt. Mit diesen Zellen wurden 50 ml Medium E + 100 µg/ml Ampicillin, die in einem 250 ml Erlenmeyerkolben vorlagen, inocculiert und in einem Luftschüttler bei 250 U/min und 39°C inkubiert. Nach zweitägiger Inkubation wurde die Bakteriensuspension verdünnt und auf LB-Agar (Lennox, 1955, Virology, 1:190) ausgestrichen, der mit 100 µg/ml Ampicillin supplementiert worden war.

**Tabelle 1**

| Substanz | Menge pro Liter | Bemerkung |
|---|---|---|
| K₂HPO₄ | 10 g | |
| NaNH₄HPO₄*4 H₂O | 3,5 g | |
| Zitronensäure | 2 g | |
| MgSO₄ * 7 H₂O | 0,2 g | |
| Glukose | 4 g | separat sterilisieren |
| Thiamin | 0,2 µg | sterilfiltrieren |

Die Plasmid-DNA einer DV9-Transformante wurde isoliert, als pNIC-1.3 bezeichnet und mittels Agarosegelelektrophorese (Sambrook et al., Molecular cloning. A laboratory manual (1989) Cold Spring Harbour Laboratory Press) und Vergleich mit Standard-DNA-Fragmenten bekannter Länge charakterisiert. Plasmid pNIC-1.3 enthält eine Insertion von 7 kbp Länge. Die Komplementationsfähigkeit von pNIC-1.3 wurde durch erneute Transformation der panD Mutante DV9 überprüft. Die erhaltenen Transformanten waren wiederum fähig, in β-Alanin-freiem Medium E unter den oben angegebenen Bedingungen zu wachsen.

Die Subklonierung des 7 kb Inserts erfolgte durch Spaltung des Plasmids pNIC-1.3 mit den Restriktionsenzymen BamHI (Pharmacia Biotech (Freiburg, Deutschland), Produktbeschreibung BamHI, Code no. 27-0868-03), EcoRI (Pharmacia Biotech (Freiburg, Deutschland), Produktbeschreibung EcoRI, Code no. 27-0884-03) und BglII (Pharmacia Biotech (Freiburg, Deutschland), Produktbeschreibung BglII, Code no. 27-0946-02) und anschließender Ligation in den entsprechend restriktionsverdauten Vektor pK18mob (Schäfer, 1994, Gene, 145:69-73). Der erhaltene Ligationsansatz wurde in die E. coli panD Mutante DV9 elektroporiert; die Selektion auf komplementierte Transformanten erfolgte wie oben beschrieben wobei die Agarplatten in diesem Fall 50 µg/ml Kanamycin enthielten. Die Plasmide von komplementierten Einzelklonen wurden isoliert und mittels Restriktionsanalysen charakterisiert. Ein EcoRI-Subklon, im Folgenden pNIC-10 genannt, mit einem ungefähr 3 kb großen DNA-Insert wurde für die folgende Sequenzanalyse ausgewählt.

### 2. Sequenzierung des panD-Gens

Für die doppelsträngige Sequenzierung des 3 kb Fragments von pNIC-10 wurde dieses mit verschiedenen Restriktionsenzymen gespalten und die Fragmente in die Plasmide pUC19 oder pK18mob subkloniert. Die zum Sequenzieren eingesetzte Plasmid-DNA wurde nach Herstellerangaben mit dem "QIAGEN Plasmid Mini kit" (Qiagen, Inc., Chatsworth, Ca., USA) isoliert und die Bestimmung der Plasmidgrößen mittels Agarosegelelektrophorese durchgeführt.

Die Sequenzierung erfolgte nach der Dideoxy-KettenabbruchMethode von Sanger et al. (Proceedings of the National Academies of Sciences USA, 74:5463-5467, 1977) mit Modifikationen nach Zimmermann et al. (Nucleic Acids Research, 18:1067, 1990). Es wurde der "Cy5-AutoRead Sequencing kit" von Pharmacia (Product No. 27-2690-02, Freiburg, Germany) angewandt. Die gelelektrophoretische Auftrennung und Analyse der Sequenzierreaktion erfolgte in einem "Long Ranger Gel Solution"-Polyacrylamidgel (FMC BioProducts, Rockland, Me., USA) mit dem "automatischen Laser-Fluoreszenz (A.L.F.) Express DNA Sequenziergerät" von Amersham Pharmacia Biotech (Uppsala, Schweden). Die erhaltenen Roh-Sequenzdaten wurden anschließend unter Anwendung des Staden-Programpakets (Nucleic Acids Research, 14:217-231, 1986) Version 97-0 prozessiert. Sämtliche Einzelsequenzen der pNIC-10 Subklone wurden zu einem zusammenhängenden 3060 bp langen Contig assembliert, der als Contig13 bezeichnet wurde. Die computergestützte Kodierbereichsanalyse mit dem Programm XNIP (Staden, 1986, Nucleic Acids Research, 14:217-231) des gesamten DNA-Fragments ergab die Identifizierung von fünf offenen Leseraster (ORFs).

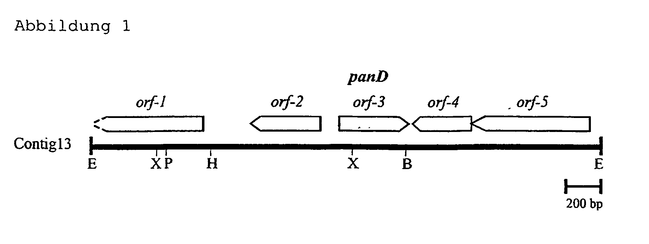

In Abbildung 1 ist eine Restriktionskarte von Contig13 sowie die Lage der als orf- bis orf-5 bezeichneten ORFs dargestellt. Homologieanalysen wurden mit den "BLAST search programs" (Gish and States, 1993, Nature of Genetics, 3:266-272; Altschul et al., 1990, Journal of Molecular Biology, 215:403-410), welche über den Online-Service des NCBI-Servers der "National Library of Medicine" (Bethesda, MD, USA) zur Verfügung gestellt wurde, durchgeführt. Die Analyse von Contig13 ergab, daß orf-3 das panD-Gen ist. Im Folgenden wird orf-3 als panD bezeichnet. Die Nukleotidsequenz des das panD-Gen tragenden DNA-Fragmentes ist als SEQ ID NO 1 wiedergegeben. Die Aminosäuresequenz des sich mit obigen Methoden ergebenden panD-Genproduktes nämlich der L-Aspartat 1-Decarboxylase ist als SEQ ID NO 2 wiedergegeben.

### Beispiel 2

### Klonierung und Sequenzierung der Gene panB und panC aus C. glutamicum

### 1. Klonierung der Gene panB und panC

Chromosomale DNA von C. glutamicum ATCC13032 wurde wie bei Schwarzer und Pühler (Bio/Technology 9 (1990) 84-87) beschrieben isoliert und mit der Restriktionsendonuklease Sau3A geschnitten. Nach gelektrophoretischer Auftrennung wurden DNA-Fragmente in einem Größenbereich von 3 bis 7 kb bzw. von 9 bis 20 kb extrahiert und nachfolgend in die singuläre BamHI Schnittstelle des Vektors pBR322 ligiert. Mit den Ligationsansätzen wurde der E. coli Stamm DH5αmcr (Grant et al., Proceedings of the National Academy of Sciences of the United States of America USA, 87 (1990) 4645-4649) transformiert (Hanahan, Journal of Molecular Biology 166 (1983) 557-580). Inserttragende Kolonien wurden anhand ihrer Tetracyclinsensitivität nach Überimpfen auf 10 µg/ml Tetracyclin enthaltende LB-Agarplatten identifiziert. Durch Plasmidpräparationen (Sambrook et al., Molecular cloning. A laboratory manual (1989) Cold Spring Harbour Laboratory Press) von vereinigten Klonen wurden 8 Gruppen, welche je 400 Plasmide mit einer Insertgröße von 9 bis 20 kb und 9 Gruppen, welche je 500 Plasmide mit einer Insertgröße von 3 bis 7 kb, enthielten isoliert. Die E. coli panB Mutante SJ2 (Cronan et al. 1982, Journal of Bacteriology 149: 916-922) wurde mit dieser Genbank mittels Elektroporation (Wehrmann et al. 1994, Microbiology 140: 3349-3356) transformiert. Die Transformationsansätze wurden direkt auf CGXII-Medium mit 15 g/l Agar (Keilhauer et al., Journal of Bacteriology (1993) 175: 5595-5603) ausplattiert. Von Klonen, welche in der Lage waren ohne Pantothenatsupplementation zu wachsen, wurde Plasmid-DNA isoliert (Sambrook et al., Molecular cloning. A laboratory manual (1989) Cold Spring Harbour Laboratory Press). Bei 8 Plasmiden konnte durch Retransformation die Fähigkeit, den panB-Defekt der E. coli Mutante SJ2 heterolog zu komplementieren, bestätigt werden.

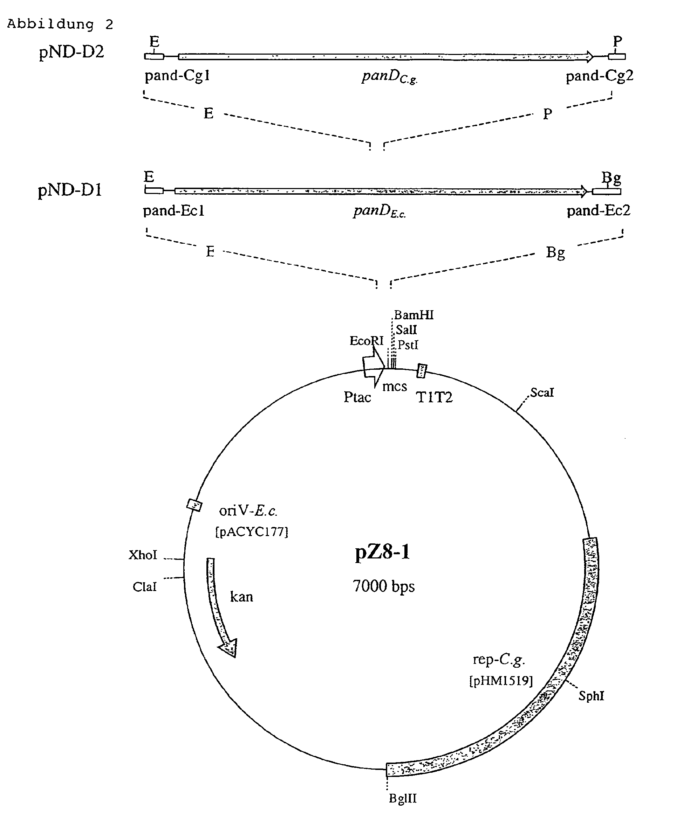

Mit diesen 8 Plasmiden wurde eine Restriktionskartierung durchgeführt. Einer der untersuchten Plasmidvektoren, im Folgendem pUR1 genannt enthielt ein Insert von 9,3 kb (Abbildung 2). Die Transformation der E. coli panC Mutante DV39 (Vallari und Rock 1985, Journal of Bacteriology 164: 136-142) ergab, daß der Vektor pUR1 ebenfalls in der Lage war den panC Defekt dieser Mutante zu komplementieren.

### 2. Sequenzierung des panB- und des panC-Gens

Ein 2,2 kb großes Fragment des Inserts (Abbildung 2) von pUR1 wurde nach der Dideoxy-Kettenabbruchmethode von Sanger et al. sequenziert (Proceedings of the National Academy of Sciences of the United States of America USA (1977) 74: 5463-5467). Hierzu wurden zunächst mittels Exonuklease III Subklone erzeugt, die mit Hilfe von Standard Primern (Universal und reverse primer der Firma Boehringer Mannheim, Deutschland) sequenziert wurden. Die gelelektrophoretische Analyse der Sequenzieransätze erfolgte mit dem automatischem Laser-Fluoreszenz Sequenziergerät (A.L.F.) von Amersham Pharmacia Biotech (Uppsala, Schweden). Die erhaltene Nukleotidsequenz wurde mit dem Programmpaket HUSAR (Release 4.0, EMBL, Cambridge, GB) analysiert. Die Nukleotidsequenz ist als SEQ ID NO 3 wiedergegeben. Die Analyse ergab die Identizierung von zwei offenen Leserastern. Ein offenes Leseraster von 813 bp Länge, das als panB-Gen identifiziert wurde, kodiert für ein Polypeptid von 271 Aminosäuren und ist als SEQ ID NO 4 wiedergegeben. Das zweite offene Leseraster, das als panC-Gen identifiziert wurde, umfaßt 837 Basenpaare. Es kodiert für ein Polypeptid von 279 Aminosäuren, das als SEQ ID NO 5 wiedergegeben ist.

### Beispiel 3

### Konstruktion von Vektoren zur Expression von panD, panBC und panDBC

Die Pantothenatbiosynthese-Gene aus C. glutamicum und E. coli wurden unter Anwendung der Polymerase-Kettenreaktion (PCR) sowie synthetischen Oligonucleotiden amplifiziert. Die PCR-Experimente wurden mit der Taq DNA polymerase der Firma Gibco-BRL (Eggestein, Deutschland) in einem "PCT-100 Thermocycler" (MJ Research Inc., Watertown, Mass., USA) durchgeführt. Auf einen einmaligen Denaturierungsschritt von 2 Minuten bei 94°C folgte ein Denaturierungsschritt von 90 Sekunden bei 94°C, ein Annealingschritt für 90 Sekunden bei einer Primerabhängigen Temperatur von T=(2AT+4GC) -5 °C (Suggs, et al., 1981, S. 683-693, In: D. D. Brown, and C. F. Fox (Eds.), Developmental biology using purified genes. Academic Press, New York, USA) sowie ein 90 Sekunden dauernder Extensionsschritt bei 72°C. Die letzten drei Schritte wurden 35 mal zyklisch wiederholt und die Reaktion wurde mit einem finalen Extensionsschritt von 10 Minuten bei 72°C beendet. Die so amplifizierten Produkte wurden, nachdem sie im Agarosegel elektrophoretisch geprüft worden sind, den Herstellerangaben zufolge in den Vektor pCR^{®}2.1 (Original TA Clonong Kit, Invitrogene (Leek, Niederlande), Produktbeschreibung Original TA Cloning^{®} Kit, Cat. no. KNM2030-01).) ligiert und anschließend in den E. coli Stamm TOP10F' transformiert. Die Selektion auf Transformanten erfolgte durch Inkubation bei 37°C für 24 Stunden auf LB-Agarplatten mit 100 µg/ml Ampicillin und 40 µg/ml X-Gal (5-Bromo-4-Chloro-3-Indolyl-β-D-Galaktosid).

Ausgehend von den Nucleotidsequenzen der Pantothenatbiosynthese-Gene panD (SEQ ID NO 1) und panBC (SEQ ID NO 3) von C. glutamicum ATCC 13032 und von E. coli K12 (W.K. Merkel and B.P. Nichols, 1993, GenBank: L17086) wurden PCR-Primer synthetisiert (MWG Biotech, Ebersberg, Deutschland). Diese Primer wurden so ausgewählt, daß die amplifizierten Fragmente die Gene sowie deren native Ribosomen-Bindestellen, nicht aber mögliche Promotor-Regionen enthalten. Zusätzlich wurden geeignete Restriktionsschnittstellen eingefügt, die das Klonieren in den Zielvektor ermöglichen. Die Sequenzen der PCR-Primer, die eingefügten Schnittstellen (Sequenz unterstrichen) sowie das amplifizierte Gen (Fragmentgröße in bp ist in Klammern angegeben) sind in der folgenden Tabelle 2 aufgelistet.

**Tabelle 2**

| Primer | Sequenz mit Restriktionsschnittstelle | Produkt | Plasmid |
|---|---|---|---|
| panD-Ec1 | | panD_{E.c.} (462 bp) | pND-D1 |
| panD-Ec2 | | | |
| panD-Cg1 | | panD_{C.g.} (405 bp) | pND-D2 |
| panD-Cg2 | | | |
| panBC-E1 | | panBC_{E.c.} (1700 bp) | pND-BC1 |
| panBC-E2 | | | |
| panBC-C1 | | panBC_{C.g.} (1700 bp) | pND-BC2 |
| panBC-C2 | | | |

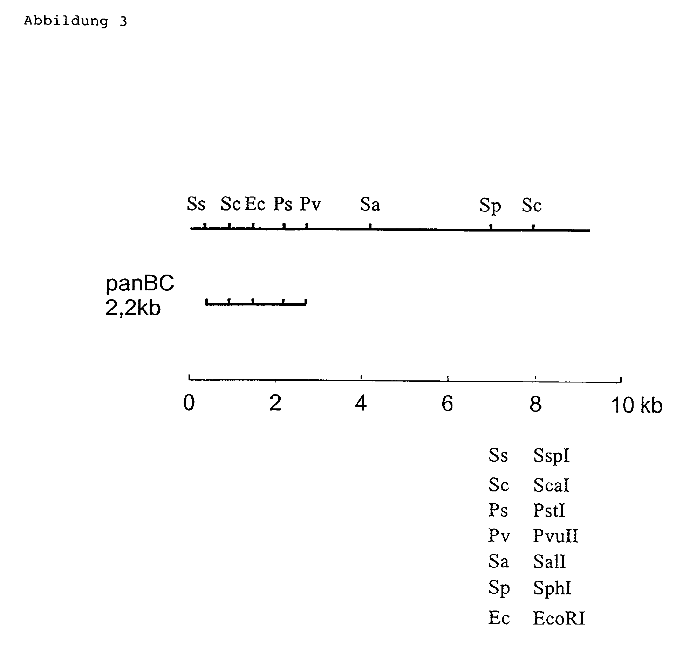

Als Basisvektor zur Expression sowohl in C. glutamicum als auch in E. coli wurde der in Abbildung 3 dargestellte E. coli-C. glutamicum-Shuttle-Expressionsvektor pZ8-1 (Europäische Patentschrift 0 375 889) eingesetzt. Die zuvor in den Vektor pCR^{®}2.1 klonierten Amplifikate wurden mittels der Primer-inserierten Restriktionsschnittstellen in den ebenso behandelten Expressionsvektor pZ8-1 ligiert und somit unter die Kontrolle des auf diesem Plasmid enthaltenen tac-Promotors gebracht. Einzige Ausnahme stellt das Amplifikat panD_{E.c.} dar, hier wurde das EcoRI-BglII-Fragment in die kompatiblen EcoRI-BamHI-Restriktionsenden des Vektors pZ8-1 kloniert. Die jeweiligen Plasmidbezeichnungen für die so konstruierten Expressionsplasmide sind in der Tabelle 2 angegeben. Der Expressionsvektor pZ8-1 mit dem Gen panD_{E.c.} von E. coli wird pND-D1 und pZ8-1 mit dem Gen panD_{C.g.} von C. glutamicum wird pND-D2 genannt. Entsprechend werden die Expressionsplasmide, die panBC_{E.c.} und panBC_{C.g.} enthalten als pND-BC1 bzw. pND-BC2 bezeichnet. Beispielhaft ist in der Abbildung 3 die Klonierungsstrategie für die Gene panD_{E.c.} und panD_{C.g.} in den Vektor pZ8-1 dargestellt. Die korrekte Klonierung aller Expressionsplasmide wurde durch Sequenzierung der jeweiligen Inserts überprüft.

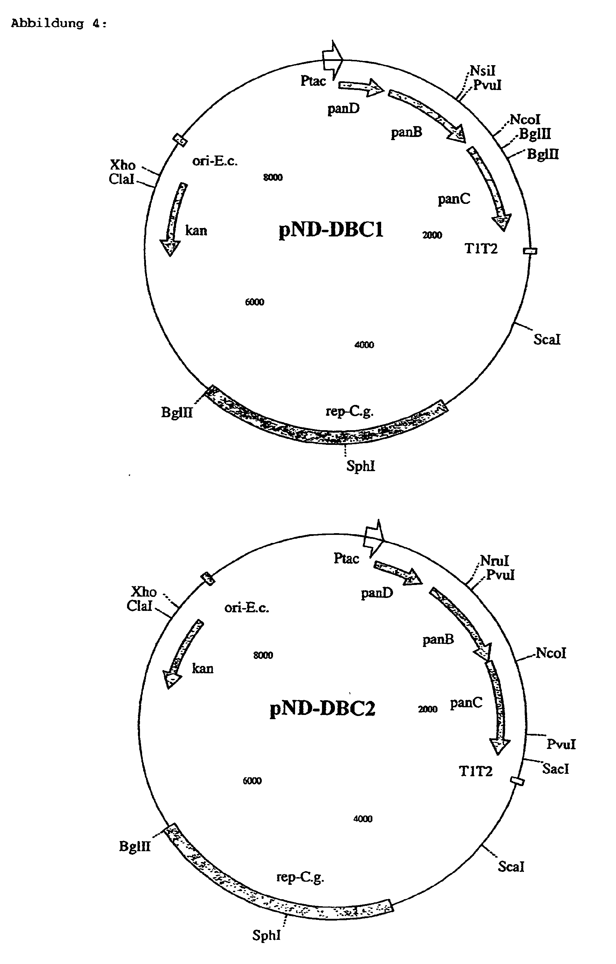

Weiterhin wurden sowohl mit den E. coli als auch mit den C. glutamicum panD-Genen ein künstliches panDBC-Operon konstruiert. Für das E. coli Operon wurde der panD_{E.c.} enthaltende Vektor pCR2.1 mit EcoRI gespalten, die DNA im Agarosegel aufgetrennt und das panD-Fragment wurde, wie schon in Beispiel 1.1 beschrieben, mittels "Nucleotrap Extraction Kit for Nucleic Acids" (Macherey und Nagel, Düren, Deutschland) aus dem Gel aufgereinigt. Anschließend wurde das Fragment in das EcoRI gespaltene Plasmid pND-BC1 ligiert. Plasmide mit einer korrekten Orientierung des panD-Gens wurden dadurch erhalten, daß das Ligationsgemisch in den panD auxothrophen E. coli Stamm DV9 transformiert und dieser wie in Beispiel 1 beschrieben auf Komplementation der Auxothrophie hin selektioniert wurde. Plasmid-DNA der komplementierten Mutanten wurde isoliert und die korrekte Genanordnung wurde durch Ansequenzierung des Inserts des pND-DBC1 genannten Plasmids bestätigt (Abbildung 4).

Für die Konstruktion des C. glutamicum panDBC-Operons wurde ähnlich verfahren. Der panD_{C.g.} enthaltene Vektor pCR2.1 wurde EcoRI gespalten, wodurch das panD_{C.g.}-Gen zum einen über die Primer-interne und zum anderen über eine EcoRI-Schnittstelle des Vektors aus diesem herausgespalten wurde. Dieses Gen-Fragment wurde nach Aufreinigung in den EcoRIgespaltenen Vektor pZ8-1 kloniert und Plasmide mit der korrekten panD-Orientierung, pND-D4 genannt, wurden wie oben beschrieben erhalten und überprüft. Anschließend wurde das Plasmid pND-D4 mit dem Restriktionsenzym SalI gespalten und mit dem aufgereinigten panBC-Fragment, welches durch SalI-Verdau (Pharmacia Biotech (Freiburg, Deutschland), Produktbeschreibung SalI Code no. 27-0882-01) des Plasmids pND-BC2 erhalten wurde, ligiert. Das Elektroporationsgemisch wurde in den E. coli Stamm DH5αMCR elektroporiert und die 10 Plasmide mit der Genanordnung panDBC wurden durch Restriktionsanalysen ermittelt. Die korrekte Genanordnung eines dieser Plasmide, das als pND-DBC2 (Abbildung 4) bezeichnet wurde, wurde durch Sequenzanalyse verifiziert.

Der Expressionsvektor pZ8-1 sowie die auf diesem Plasmid beruhenden Konstrukte pND-D1, pND-D2 und pND-DBC1 wurden in den E. coli Stamm MG1655 transformiert und Transformanten auf LB-Agar (Lennox, 1955, Virology, 1:190) + 50 µg/ml Kanamycin selektioniert. Die erhaltenen Stämme wurden MG1655/pZ8-1, MG1655/pND-D1, MG1655/pND-D2 und MG1655/pND-DBC1 genannt.

Durch Elektroporation der Plasmide pZ8-1, pND-D1, pND-D2 und pND-DBC2 in den C. glutamicum Stamm ATCC13032 und anschließender Selektion auf LB-Agar (Lennox, 1955, Virology, 1:190) + 25 µg/ml Kanamycin wurden die Stämme ATCC13032/pZ8-1, ATCC13032/pND-D1, ATCC13032/pND-D2 und ATCC13032/pND-DBC2 erhalten.

### Beispiel 4

### Bildung von Pantothenat durch verschiedene E. coli K12 Stämme

Die quantitative Bestimmung von D-Pantothenat erfolgte mittels des Lactobacillus plantarum Pantothenat-Assays (Teststamm: Lactobacillus plantarum ATCC 8014, Cat. No.3211-30-3; Kulturmedium: Bacto Pantothenate Assay Medium (DIFCO Laboratories, Michigan, USA), Cat. No. 0604-15-3) Dieser Indikatorstamm kann nur bei Anwesenheit von Pantothenat im angegebenen Kulturmedium wachsen und zeigt eine photometrisch meßbare, lineare Abhängigkeit des Wachstums von der Pantothenat-Konzentration des Mediums. Für die Kalibrierung wurde das Hemicalciumsalz von Pantothenat eingesetzt (Sigma, Produktbezeichnung P 2250). Die optische Dichte wurde an einem LKB Biochrom Photometer der Firma Pharmacia Biotech (Freiburg, Deutschland) bei einer Messwellenlänge von 580 nm (o.D.₅₈₀)bestimmt.

Für die Pantothenat-Produktion der E. coli Stämme MG1655/pZ8-1, MG1655/pND-D1, MG1655/pND-D2 und MG1655/pND-DBC1 wurden 50 ml Testmedium (Medium E mit 50 µg/ml Kanamycin) aus einer 16 Stunden alten Kultur des gleichen Mediums mit einer o.D.₅₈₀ von 0,1 angeimpft. Nach 5 und 72stündiger Inkubation dieser Kulturen bei 37°C und 250 U/min wurden die Zellen durch 10minütige Zentrifugation bei 5000 x g pelletiert. Der erhaltene zellfreie Überstand wurde sterilfiltriert und bis zur Pantothenat-Quantifizierung bei 4°C gelagert.

Die Quantifizierung des D-Pantothenats im Kulturüberstand erfolgte mittels L. plantarum ATCC 8014 nach Angaben des Handbuchs der Firma DIFCO (DIFCO MANUAL, 10th Edition, S. 1100-1102; Michigan, USA). Die Ergebnisse dieser Messungen sind in der Tabelle 3 dargestellt.

**Tabelle 3**

| Stamm | Gen | oD₅₈₀ und Pantothenat-Akkumulation (µg/ml) | | | |
|---|---|---|---|---|---|
| | | 5 Std. | | 72 Std. | |
| | | oD₅₈₀ | Pan. | oD₅₈₀ | Pan. |
| MG1655/pZ8-1 | - | 2,0 | 0,30 | 2,3 | 1,47 |
| MG1655/pND-D1 | panD_{E.c.} | 2,3 | 0,90 | 2,5 | 6,95 |
| MG1655/pND-DBC1 | panDBC_{E.c.} | 2,0 | 0,96 | 2,0 | 6,96 |
| MG1655/pND-D2 | panD_{C.g.} | 2,2 | 4,07 | 2,3 | 9,66 |

### Beispiel 5

### Bildung von Pantothenat durch verschiedene Stämme von C. glutamicum

Die Bildung von Pantothenat durch die C. glutamicum Stämme ATCC13032/pZ8-1, ATCC13032/pND-D1, ATCC13032/pND-D2 und C. glutamicum ATCC13032/pND-DBC2 wurden in Medium CGXII (Keilhauer et al., 1993, Journal of Bacteriology, 175:5595-5603; Tabelle 4), das mit 25 µg/ml Kanamycin supplementiert wurde, geprüft. Dieses Medium wird im Folgenden als C. glutamicum-Testmedium bezeichnet. Je 50 ml C. glutamicum-Testmedium wurden aus einer 16 Stunden alten Kultur des gleichen Mediums mit einer o.D.₅₈₀ von 0,1 angeimpft. Nach 48stündiger Inkubation bei 30°C und 150 U/min wurden die Zellen durch 10minütige Zentrifugation bei 5000 x g entfernt, der Überstand sterilfiltriert und die Pantothenat-Konzentration wie in Beispiel 4 beschrieben bestimmt. Die Ergebnisse der Pantothenat-Produktion durch die verschiedenen Stämme von C. glutamicum sind in Tabelle 5 zusammengefasst.

**Tabelle 4**

| Substanz | Menge pro Liter | Bemerkung |
|---|---|---|
| (NH4)₂ SO₂ | 20 g | |
| Harnstoff | 5 g | |
| KH₂PO₄ | 1 g | |
| K₂HPO₄ | 1 g | |
| MgSO₄ * 7 H₂O | 0.25 g | |
| MOPS | 42 g | |
| CaCl₂ | 10 mg | |
| FeSO₄ * 7 H₂O | 10 mg | |
| MnSO₄ * H₂O | 10 mg | |
| ZnSO₄ * 7 H₂O | 1 mg | |
| CuSO₄ | 0.2 mg | |
| NiCl₂ * 6 H₂O | 0.02 mg | |
| Biotin | 0.5 mg | |
| Glukose | 40 g | separat autoklavieren |
| Protocatechusäure | 0.03 mg | sterilfiltrieren |

**Tabelle 5**

| Stamm | Gen | Pantothenat (µg/ml) | |
|---|---|---|---|
| | | oD₅₈₀ | Pan. |
| ATCC13032/pZ8-1 | - | 21 | 0,19 |
| ATCC13032/pND-D1 | panD_{E.c.} | 20 | 0,32 |
| ATCC13032/pND-D2 | panD_{C.g.} | 19 | 1,78 |
| ATCC13032/pND-DBC2 | panDBC_{C.g.} | 20 | 2,60 |

### Abbildungen

Folgende Abbildungen sind beigefügt:
- Abbildung 1: Karte des Contig13 mit orf-1-orf-5
- Abbildung 2: Karte des in pUR1 enthaltenen, klonierten DNA-Fragmentes und Lageangabe des sequenzierten DNA-Abschnittes
- Abbildung 3: Karte der Plasmide pZ8-1, pND-D1 und pND-D2
- Abbildung 4: Karte der Plasmide pND-DBC1 und pND-DBC2

Die in den Abbildungen verwendeten Abkürzungen haben folgende Bedeutung:
- rrnBT1T2:: Transkriptions-Terminator des rrnB-Gens
- Ptac:: tac Promotor
- panB:: Kodierbereich des panB Gens
- panC:: Kodierbereich des panC Gens
- panD:: Kodierbereich des panD Gens
- rep-C.g.:: DNA-Region für Replikation in C. glutamicum
- oriV-E.c.:: Ursprung für vegetativen Transfer in E. coli
- kan:: Resistenzgen für Kanamycin
- EcoRI:: Schnittstelle des Restriktionsenzyms EcoRI
- E:: Schnittstelle des Restriktionsenzyms EcoRI
- BamHI:: Schnittstelle des Restriktionsenzyms BamHI
- B: Schnittstelle des Restriktionsenzyms BamHI
- BglII:: Schnittstelle des Restriktionsenzyms BglII
- ClaI:: Schnittstelle des Restriktionsenzyms ClaI
- H:: Schnittstelle des Restriktionsenzyms HindIII
- NcoI:: Schnittstelle des Restriktionsenzyms NcoI
- NruI:: Schnittstelle des Restriktionsenzyms NruI
- NsiI:: Schnittstelle des Restriktionsenzyms NsiI
- P:: Schnittstelle des Restriktionsenzyms PstI
- PstI:: Schnittstelle des Restriktionsenzyms PstI
- PvuI:: Schnittstelle des Restriktionsenzyms PvuI
- SacI:: Schnittstelle des Restriktionsenzyms SacI
- SalI:: Schnittstelle des Restriktionsenzyms SalI
- ScaI:: Schnittstelle des Restriktionsenzyms ScaI
- SphI:: Schnittstelle des Restriktionsenzyms SphI
- X:: Schnittstelle des Restriktionsenzyms XbaI
- XhoI:: Schnittstelle des Restriktionsenzyms XhoI

## Patentansprüche

1. PanD-Gen, das eine für Aspartat-1-decarboxylase codierende Nucleotidsequenz enthält, ausgewählt aus der Gruppe, bestehend aus:
a) der Nukleotidsequenz, gezeigt in SEQ.-ID.-Nr. 1, oder
b) einer Nukleotidsequenz, die der Sequenz (i) innerhalb des Bereichs der Degeneration des genetischen Codes entspricht;

2. Aspartat-1-decarboxylase mit einer Aminosäuresequenz gemäß SEQ ID NO: 2.

3. Mikroorganismen der Gattungen Corynebacterium oder Escherichia in denen man das für die L-Aspartat-1-decarboxylase codierende panD-Gen gemäß Anspruch 1 überexprimiert, wobei die Kopienzahl des panD-Gens durch Transformation einem mit dieses Gen tragenden Plasmidvektor oder durch chromosomale Amplifikation erhöht, vorliegt.

4. Mikroorganismen gemäß Anspruch 3, in denen zusätzlich das für die Ketopantoathydroxymethyltransferase kodierende panB-Gen und das für die Pantothenatsynthetase kodierende panC-Gen gemäß der SEQ ID NO: 3 überexprimiert vorliegt.

5. Vektor enthaltend DNA mit der Nukleotidsequenz, gezeigt in SEQ ID NO: 1 und gegebenenfalls die Nukleotidsequenz, gezeigt in SEQ ID NO: 3.

6. Plasmidvektor gemäß Anspruch 5, hinterlegt in Corynebacterium glutamicum ATCC 13032/pND-D2 unter der Bezeichnung DSM 12438.

7. Plasmidvektor gemäß Anspruch 5, hinterlegt in Corynebacterium glutamicum ATCC 13032/pND-DBC2 unter der Bezeichnung DSM12437.

8. Mikroorganismen gemäß Anspruch 3, in die zur Erzielung der Überexpression stromaufwärts des Strukturgens Expressionskassetten eingebaut sind.

9. Mikroorganismen gemäß den Anspruch 3, die weitere Metabolit- bzw. Antimetabolit-Resistenzmutationen aufweisen.

10. Mikroorganismen gemäß den Ansprüche 3, 8 und 9, in denen die Stoffwechselwege zumindest teilweise ausgeschaltet sind, die die Pantothenat-(Pantothensäure)-bildung verringern.

11. Mikroorganismen gemäß den Ansprüchen 3, 8 bis 10, in denen man zusätzlich zum panD-Gen die übrigen Gene des Stoffwechselweges der Pantothensäurebildung, einzeln oder gemeinsam überexprimiert.

12. Mikroorganismen gemäß den Ansprüchen 3, 8 bis 11, in denen man eines der Gene, die für die Enzyme Ketopantoat-Hydroxymethyltransferase (EC 4.1.2.12), und Pantothenat-Synthetase (EC 6.3.2.1) kodieren, mit der Herkunft Corynebacterium überexprimiert.

13. Mikroorganismen gemäß den Ansprüchen 11 und 12, mit verschiedenen kompatiblen, die genannten Gene einzeln enthaltenden Plasmidvektoren transformiert sind.

14. Mikroorganismen gemäß den Ansprüchen 1, 11 und 12, welche einen Plasmidvektor enthalten und der Plasmidvektor eines oder mehrere der genannten Gene einschließlich des panD-Gens trägt, in dem die Gene nacheinander angeordnet und unter die Kontrolle eines gemeinsamen Promotors oder getrennt voneinander angeordnet unter die Kontrolle verschiedener Promotoren gestellt werden.

15. Verfahren zur Herstellung von Pantothensäure, bei dem man folgende Schritte durchführt:
a) Fermentation von Mikroorganismen gemäß einem oder mehreren der Ansprüche 3, 4, 8 bis 14,
b) Anreicherung der Pantothensäure im Medium oder in den Zellen der Mikroorganismen, und
c) Isolieren der Pantothensäure

16. Verfahren gemäß Anspruch 15, bei dem man die überexprimierten Gene aus Mikroorganismen der Gattung Corynebacterium stammen.

17. Verfahren gemäß den Ansprüchen 15 oder 16, bei dem man in Stufe a) eine Vorstufe der Pantothensäure zusetzt, ausgewählt aus der Gruppe Aspartat, β-Alanin, Ketoisovalerat, Ketopantoat oder Pantoat.

18. Stamm von Corynebacterium glutamicum ATCC 13032/pND-D2 hinterlegt als DSM 12438 bei der DSMZ, Braunschweig.

19. Stamm von Corynebacteriumglutamicum ATCC 13032/pND-DBC2 hinterlegt als DSM 12437 bei der DSMZ, Braunschweig.

## Claims

1. PanD gene comprising a nucleotide sequence coding for aspartate 1-decarboxylase, which sequence is selected from the group consisting of:
a) the nucleotide sequence depicted in SEQ ID No 1, or
b) a nucleotide sequence corresponding to the sequence (i) within the range of degeneracy of the genetic code.

2. Aspartate 1-decarboxylase having an amino acid sequence according to SEQ ID NO: 2.

3. Microorganisms of the genus Corynebacterium or Escherichia, in which the panD gene according to Claim 1, which codes for L-aspartate 1-decarboxylase, is overexpressed, the panD gene copy number having been increased by transformation with a plasmid vector carrying this gene or by chromosomal amplification.

4. Microorganisms according to Claim 3, in which additionally the panB gene coding for ketopantoate hydroxymethyltransferase and the panC gene coding for pantothenate synthetase according to SEQ ID NO: 3 are overexpressed.

5. Vector comprising DNA containing the nucleotide sequence depicted in SEQ ID NO: 1 and optionally the nucleotide sequence depicted in SEQ ID NO: 3.

6. Plasmid vector according to Claim 5, deposited in Corynebacterium glutamicum ATCC 13032/pND-D2 under the name DSM 12438.

7. Plasmid vector according to Claim 5, deposited in Corynebacterium glutamicum ATCC 13032/pND-DBC2 under the name DSM 12437.

8. Microorganisms according to Claim 3, into which expression cassettes have been incorporated upstream of the structural gene in order to achieve overexprssion.

9. Microorganisms according to Claim 3, which have further metabolite or anti-metabolite resistance mutations.

10. Microorganisms according to Claims 3, 8 and 9, in which at least some of the metabolic pathways that reduce the formation of pantothenate (pantothenic acid) have been switched off.

11. Microorganisms according to Claims 3, 8 to 10, in which, in addition to the panD gene, the remaining genes of the metabolic pathway of the formation of pantothenic acid are overexpressed individually or together.

12. Microorganisms according to Claims 3, 8 to 11, in which either of the genes coding for the enzymes ketopantoate hydroxymethyltransferase (EC 4.1.2.12) and pantothenate synthetase (EC 6.3.2.1) and origninating from Corynebacterium are overexpressed.

13. Microorganisms according to Claims 11 and 12, transformed with various compatible plasmid vectors containing said genes in each case individually.

14. Microorganisms according to Claims 1, 11 and 12, which harbour a plasmid vector carrying one or more of said genes, including the panD gene, in which vector the genes are arranged one after the other and put under the control of a common promoter, or are arranged separately from one another and put under the control of different promoters.

15. Method for producing pantothenic acid, which comprises the following steps:
a) fermentation of microorganisms according to one or more of Claims 3, 4, 8 to 14;
b) concentration of said pantothenic acid in the medium or in the cells of said microorganisms; and
c) isolation of said pantothenic acid.

16. Method according to Claim 15, in which the overexpressed genes are from microorganisms of the genus Corynebacterium.

17. Method according to Claim 15 or 16, which comprises adding, in step a), a precursor of pantothenic acid, selected from the group consisting of aspartate, β-alanine, ketoisovalerate, ketopantoate and pantoate.

18. Strain of Corynebacterium glutamicum ATCC 13032/pND-D2, deposited as DSM 12438 with the DSMZ, Brunswick, Germany.

19. Strain of Corynebacterium glutamicum ATCC 13032/pND-DBC2, deposited as DSM 12437 with the DSMZ, Brunswick, Germany.

## Revendications

1. Gène panD, qui contient une séquence nucléotidique codant pour l'aspartate-1-décarboxylase, choisi dans le groupe constitué par :
a) la séquence nucléotidique présentée dans SEQ ID n° 1, et
b) une séquence nucléotidique qui correspond à la séquence (i) dans le cadre de la dégénérescence du code génétique ;

2. Aspartate-1-décarboxylase ayant une séquence d'aminoacides selon SEQ ID n° 2.

3. Micro-organismes appartenant au genre *Corynebacterium* ou *Escherichia*, dans lesquels le gène panD codant pour la L-aspartate-1-décarboxylase selon la revendication 1, est présent surexprimé, le nombre de copies du gène panD étant augmenté par transformation d'un vecteur plasmidique portant ce gène ou par amplification chromosomique.

4. Micro-organismes selon la revendication 3, dans lesquels en outre le gène panB codant pour la cétopantoate hydroxyméthyltransférase et le gène panC codant pour la pantothénate synthétase selon la séquence SEQ ID n° 3 sont présents surexprimés.

5. Vecteur contenant de l'ADN ayant la séquence nucléotidique présentée dans SEQ ID n° 1 et éventuellement la séquence nucléotidique présentée dans SEQ ID n° 3.

6. Vecteur plasmidique selon la revendication 5, déposé dans *Corynebacterium glutamicum* ATCC 13032/pND-D2 sous la désignation DSM 12438.

7. Vecteur plasmidique selon la revendication 5, déposé dans *Corynebacterium glutamicum* ATCC 13032/pND-DBC2 sous la désignation DSM 12437.

8. Micro-organismes selon la revendication 3, dans lesquels, pour l'obtention de la surexpression, des cassettes d'expression sont insérées en amont du gène structural.

9. Micro-organismes selon la revendication 3, qui présentent d'autres mutations de résistance à des métabolites ou des antimétabolites.

10. Micro-organismes selon les revendications 3, 8 et 9, dans lesquels les voies métaboliques qui diminuent la formation de pantothénate (acide pantothénique) sont au moins partiellement interrompues.

11. Micro-organismes selon les revendications 3, 8 à 10, dans lesquels en plus du gène panD on surexprime, individuellement ou ensemble, les autres gènes de la voie métabolique de la formation de l'acide pantothénique.

12. Micro-organismes selon les revendications 3, 8 à 11, dans lesquels on surexprime l'un des gènes qui codent pour les enzymes cétopantoate hydroxyméthyltransférase (EC 4.1.2.12) et pantothénate synthétase (EC 6.3.2.1) ayant pour origine *Corynebacterium*.

13. Micro-organismes selon les revendications 11 et 12, transformés par divers vecteurs plasmidiques compatibles, contenant individuellement les gènes cités.

14. Micro-organismes selon les revendications 1, 11 et 12, qui contiennent un vecteur plasmidique et le vecteur plasmidique porte un ou plusieurs des gènes cités, y compris le gène panD, dans lequel les gènes sont placés les uns derrière les autres et sous le contrôle d'un promoteur commun ou, placés séparément les uns des autres, sont mis sous le contrôle de promoteurs différents.

15. Procédé pour la production d'acide pantothénique, dans lequel on effectue les étapes suivantes :
a) culture par fermentation de micro-organismes selon une ou plusieurs des revendications 3, 4, 8 à 14,
b) accumulation de l'acide pantothénique dans le milieu ou dans les cellules des micro-organismes, et
c) isolement de l'acide pantothénique.

16. Procédé selon la revendication 15, dans lequel les gènes surexprimés proviennent de micro-organismes appartenant au genre *Corynebacterium*.

17. Procédé selon la revendication 15 ou 16, dans lequel on ajoute dans l'étape a) un précurseur de l'acide pantothénique, choisi dans le groupe constitué par l'aspartate, la β-alanine, le céto-isovalérate, le cétopantoate et le pantoate.

18. Souche de *Corynebacterium glutamicum* ATCC 13032/pND-D2, déposée en tant que DSM 12438 à la DSMZ (Deutsche Sammlung von Mikroorganismen und Zellkulturen), Braunschweig.

19. Souche de *Corynebacterium glutamicum* ATCC 13032/pND-DBC2, déposée en tant que DSM 12437 à la DSMZ, Braunschweig.
